# EUROPÄISCHE PATENTSCHRIFT

(11) **EP 4 008 709 B1**
(45) Veröffentlichungstag und Bekanntmachung des Hinweises auf die Patenterteilung: **01.05.2024**
(21) Anmeldenummer: 20211797.4
(22) Anmeldetag: 04.12.2020
(51) Int. Cl.: C07C 45/50, C07C 47/347

(54) **VERFAHREN ZUR HERSTELLUNG POLYZYKLISCHER ALIPHATISCHER DIALDEHYDE**
METHOD FOR THE PREPARATION OF POLYCYCLIC ALIPHATIC DIALDEHYDES
PROCÉDÉ DE PRODUCTION DE DIALDÉHYDE ALIPHATIQUE POLYCYCLIQUE

(43) Veröffentlichungstag der Anmeldung: 08.06.2022
(73) Patentinhaber: OQ Chemicals GmbH, 40789 Monheim am Rhein (DE)
(72) Erfinder: Vogelsang, Dennis, 48249 Dülmen (DE)
(74) Vertreter: Michalski Hüttermann & Partner Patentanwälte mbB

(56) Entgegenhaltungen:
- EP-A1- 1 529 769
- EP-A1- 1 529 771
- DE-A1-102006 004 318

## Beschreibung

Die vorliegende Erfindung betrifft ein Verfahren zur Herstellung polyzyklischer aliphatischer Dialdehyde durch Hydroformylierung polyzyklischer aliphatischer Diolefine in Gegenwart von Synthesegas an einem Organophosphorliganden modifizierten Metallkatalysatorsystem mit einem Übergangsmetall der 8. - 10. Nebengruppe, wobei die Hydroformylierung mittels eines wasserlöslichen Diphosphin- oder Triarylphosphin-Komplexkatalysators bei einem Druck von größer oder gleich 0,5 MPa und kleiner oder gleich 10 MPa und einer Temperatur von größer oder gleich 70°C und kleiner oder gleich 150°C in einer homogenen flüssigen Reaktionsphase durchgeführt wird, wobei die homogene flüssige Phase mindestens ein nicht-wässriges Lösungsmittel, Diolefin und/oder deren Mono- und/oder Dialdehyde als Reaktionsprodukte und eine wässrige Katalysatorlösung umfasst, wobei die Mengenverhältnisse dieser Komponenten in der Lösung so gesteuert werden, dass unter den Reaktionsbedingungen eine einphasige Lösung vorliegt.

Ungesättigte zyklische Kohlenwasserstoffe mit mindestens zwei isolierten Doppelbindungen in der Ringstruktur stellen wertvolle chemische Ausgangsprodukte dar, welche sich aufgrund ihrer speziellen Geometrie zu funktional außergewöhnlichen Verbindungen umsetzen lassen. Das ringförmig aufgebaute Kohlenwasserstoffgerüst verleiht dabei den Molekülen besondere Eigenschaften, welches sich sowohl in speziellen Umsetzungsbedingungen wie auch in besonderen Anwendungsfunktionen widerspiegelt. Einige wichtige Vertreter für diese Verbindungsklasse sind beispielsweise das Cyclopentadien und das durch Dimerisierung von Cyclopentadien zugängliche und großtechnisch herstellbare Dicyclopentadien (DCP, nach IUPAC Tricyclo[5.2.1.0^{2,6}]deca-3,8-dien).

Auch die weitere Funktionalisierung dieser Verbindungen durch Umsetzung einer oder mehrerer Doppelbindungen zu anderen funktionalen Gruppen ist bekannt und in der Literatur beschrieben. So können die Doppelbindungen beispielsweise im Rahmen einer Hydroformylierung durch katalytische Anlagerung von Kohlenmonoxid und Wasserstoff zu Aldehyden umgesetzt werden. Während diese Umsetzung früher nahezu ausschließlich mit Kobaltkatalysatoren durchgeführt wurde, arbeiten moderne Verfahren mit Metallkatalysatorsystemen basierend auf Übergangsmetallen der 8. - 10. Nebengruppe. Diese Katalysatoren werden als Funktion der speziell gewählten Reaktionsbedingungen als solche allein oder zusammen mit komplexbildenden Liganden, beispielsweise organischen Phosphinen oder Estern der phosphorigen Säure, eingesetzt. Unter den Reaktionsbedingungen wirksam sind nach übereinstimmender Auffassung der Fachwelt Hydridocarbonylverbindungen des Metalls M, die sich beispielsweise für Rhodium durch die allgemeine Formel H[Rh(CO)_{4-X}L_{X}] wiedergeben lassen, wobei L einen organischen Liganden bezeichnet und x gleich 0 oder eine ganze Zahl von 1 bis 3 ist. Als Umsetzungsprodukte erhält man im Falle der Umsetzung von DCP entweder das Mono- oder Dialdehyd, wobei letzteres durch einen abschließenden Hydrierschritt weiter zu den wirtschaftlich wichtigen Diol Tricyclo[5.2.1.0^{2,6}]decanedimethanol umgesetzt werden kann.

Auch in der Patentliteratur sind einige Hydroformylierungsverfahren zur Umsetzung zyklischer Olefine zu den entsprechenden Aldehyden und gegebenenfalls einer weiteren Umsetzung der Aldehyde zu den entsprechenden Alkoholen angegeben.

So beschreibt beispielsweise die DE 10 2006 004 318 A1 Verfahren zur Herstellung von 3(4),7(8)-Dihydroxymethyl-bicyclo[4.3.0]nonan durch Hydroformylierung von Bicyclo[4.3.0]nona-3,7-dien mit nachfolgender Hydrierung, wobei man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160°C und Drücken von 5 bis 35 MPa mit Synthesegas umsetzt und das so erhaltene 3(4),7(8)-Bisformyl-bicyclo[4.3.0]nonan anschließend zum 3(4),7(8)-Dihydroxymethyl-bicyclo[4.3.0]nonan hydriert.

Auch die EP 1 529 771 A1 offenbart ein Verfahren zur Herstellung von 8(9)-Formyl-tricyclo[5.2.1.0²⁶]dec-3-en durch Hydroformylierung von Dicyclopentadien in einem heterogenen Reaktionssystem unter Verwendung einer wässrigen Lösung, wasserlösliche organische Phosphor(III)verbindungen in komplexer Bindung enthaltender Übergangsmetallverbindungen der Gruppe VIII des Periodensystems der Elemente bei Temperaturen von 70 bis 150°C und Drücken von 0,5 bis 10 MPa mit Synthesegas, wobei man als wasserlösliche organische Phosphor(III)-Verbindungen spezielle sulfonierte Triarylphosphine benutzt.

Des Weiteren beschreibt die WO 2004 024 661 A1 ein Verfahren zur katalytischen Hydroformylierung olefinisch ungesättigter Verbindungen mit 3 bis 24 Kohlenstoffatomen, wobei als Katalysator zumindest ein Metall der 8. bis 10. Gruppe des Periodensystems der Elemente aufweisender, unmodifizierter Katalysator eingesetzt wird, wobei die Hydroformylierung in Gegenwart eines cyclischen Kohlensäureesters der folgenden Formel 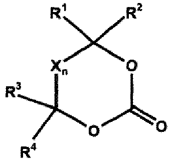
- R1, R², R³, R⁴:: jeweils gleich oder verschieden: H, substituierte oder unsubstituierte, aliphatische, alicyclische, aromatische, aliphatisch-alicyclische, aliphatisch-aromatische, alicyclisch-aromatischeKohlenwasserstoffreste mit 1 bis 27 C-Atomen.
- n:: 0-5
- X:: zweiwertiger substituierter oder unsubstituierter, aliphatischer, alicyclischer, aromatischer, aliphatisch-alicyclischer, aliphatisch-aromatischer Kohlenwasserstoffrest mit 1 bis 27 C-Atomen.
durchgeführt wird, wobei der Anteil des Kohlensäureesters zumindest 1 Gew.-% des Reaktionsgemisches beträgt. Ein weiteres Verfahren wird zudem in der EP 1 529 769 A1 beschrieben.

Derartige aus dem Stand der Technik bekannte Lösungen können noch weiteres Verbesserungspotential bieten, insbesondere hinsichtlich der Effizienz der Umsetzung sowie der Standzeiten des eingesetzten Katalysatormaterials.

Es ist daher die Aufgabe der vorliegenden Erfindung, die aus dem Stand der Technik bekannten Nachteile zumindest teilweise zu überwinden. Es ist insbesondere die Aufgabe der vorliegenden Erfindung ein Verfahren bereitzustellen, welches mit geringen Katalysatorkonzentrationen über lange Reaktionszeiträume, unter sehr moderaten Reaktionsbedingungen hohe Umsätze und Selektivitäten liefert.

Die Lösung der Aufgabe erfolgt durch die Merkmale des unabhängigen Verfahrensanspruchs. Bevorzugte Ausgestaltungen der Erfindung sind in den Unteransprüchen, in der Beschreibung oder den Figuren angegeben, wobei weitere in den Unteransprüchen oder in der Beschreibung oder den Figuren beschriebene oder gezeigte Merkmale einzeln oder in einer beliebigen Kombination einen Gegenstand der Erfindung darstellen können, wenn sich aus dem Kontext nicht eindeutig das Gegenteil ergibt.

Erfindungsgemäß gelöst wird die Aufgabe durch ein Verfahren zur Herstellung polyzyklischer aliphatischer Dialdehyde durch Hydroformylierung polyzyklischer aliphatischer Diolefine in Gegenwart von Synthesegas an einem Organophosphorliganden modifizierten Metallkatalysatorsystem mit einem Übergangsmetall der 8. - 10. Nebengruppe, wobei die Hydroformylierung mittels eines wasserlöslichen Diphosphin- oder Triarylphosphin-Komplexkatalysators bei einem Druck von größer oder gleich 0,5 MPa und kleiner oder gleich 10 MPa und einer Temperatur von größer oder gleich 70 °C und kleiner oder gleich 150 °C in einer homogenen flüssigen Reaktionsphase durchgeführt wird, wobei die homogene flüssige Phase mindestens ein nicht-wässriges Lösungsmittel, Diolefin und/oder deren Mono- und/oder Dialdehyde als Reaktionsprodukte und eine wässrige Katalysatorlösung umfasst, wobei die Massenverhältnisse dieser Komponenten in der Lösung so gesteuert werden, dass unter den Reaktionsbedingungen eine einphasige Lösung vorliegt.

Überraschenderweise wurde gefunden, dass eine Hydroformylierung polyzyklischer Diene zu den entsprechenden Dialdehyden unter moderaten Prozessbedingen hoch effizient mit einem besonders stabilen und selektiven Katalysatorsystem auch für längere Prozesszeiten durchgeführt werden kann. Die Grundlage für diese Vorteile bildet, dass die Umsetzung in einer homogenen flüssigen Phase durchgeführt wird, welche sowohl organische wie auch wässrige Komponenten umfasst. Durch die Anpassung zumindest der Mengen der angegebenen Bestandteile Katalysatorlösung, Edukt bzw. daraus entstehender Zwischen- oder Endprodukte und Lösungsmittel kann die Gesamtlösung in einem einphasigen Bereich des Phasendiagramms gehalten werden, wobei überraschenderweise der wasserlösliche Katalysator in dieser Lösung homogen verteilt vorliegt und wohl ungehindert von den Edukten und dem Synthesegas kontaktiert werden kann. Innerhalb des homogen einphasigen Bereiches liegen nicht zwei getrennte, beispielsweise eine wässrige Phase umfassend die Katalysatorlösung, und eine davon getrennte organische Phase mit den sich bildenden Produkten oder den Zwischenprodukten, sondern nur eine einzige, homogen gemischt wässrige/organische Phase vor. Durch die Steuerung der Mengen der unterschiedlichen, zumindest teilweise mischbaren Einzelbestandteile lässt sich die gesamte Umsetzung sicher einphasig durchführen und dies führt überraschenderweise dazu, dass die Umsetzung nicht auf der Stufe des Monoaldehyds stehenbleibt, sondern bis zum Dialdehyd hoch selektiv und mit einer hohen Reaktionsgeschwindigkeit erfolgt. Dieses Ergebnis ist überraschend, da wasserlösliche Katalysatoren in einer "zweiphasigen" Reaktionsumgebung nur zu einer einfachen Umsetzung führen und die Reaktion auf der Stufe des Monoaldehyds "stehenbleibt". Betrachtet man hingegen Umsetzungen in rein organischer Phase, also ohne Zusatz wässriger oder wasserlöslicher Komponenten, so können sich hohe Umsätze unter guten Selektivitäten ergeben. In Summe sollte sich aus diesen beiden unterschiedlichen Verfahren des Standes der Technik herleiten lassen, dass der Zutritt der gasförmigen Reaktionskomponenten und die Umsetzung am Katalysator unter Gegenwart wässriger Zusätze oder insgesamt der Einsatz wasserlöslicher Katalysatoren eher zu einer Verschlechterung der Synthese führen sollte. Letzteres gilt insbesondere für die Umsetzung zyklischer Diene mit einem starren Ringgerüst, welches eine effiziente Anlagerung an die Katalysatorsysteme wesentlich erschwert. Eine solche Verschlechterung ergibt sich aber auch bei den hier betrachteten herausfordernden Edukten überraschenderweise nicht. Des Weiteren scheint die Kombination aus wasserlöslichen Katalysatoren in Verbindung mit einer homogen organisch/wässriger Phase zu einer besonderen Schonung des Katalysatorsystems in der Umsetzung beizutragen, sodass vorteilhafterweise mit sehr geringen Katalysatormengen gearbeitet werden kann, welche zudem noch lange Standzeiten aufweisen.

Das erfindungsgemäße Verfahren ist ein Verfahren zur Herstellung polyzyklischer aliphatischer Dialdehyde. Polyzyklische aliphatische Dialdehyde weisen mindestens zwei nicht aromatische Ringstrukturen und insgesamt mindestens zwei Aldehydgruppen auf, wobei mindestens eine der Aldehydgruppen direkt an der Ringstruktur angeordnet ist. Bevorzugt können auch beide Aldehydgruppen direkt mit der Ringstruktur verbunden sein. Das gesamte aliphatische Ringgerüst kann beispielsweise 5 bis 20 C-Atome aufweisen und das Molekulargewicht der herstellbaren Dialdehyde kann beispielsweise zwischen 50 g/mol und 500 g/mol betragen. Mögliche Vertreter dieser Dialdehyd-Gruppe können beispielsweise Tricyclodecandicarbaldehyd oder Dicylopentandicarbaldehyd sein.

Die Umsetzung der Edukte zu den Produkten erfolgt durch Hydroformylierung polyzyklischer aliphatischer Diolefine in Gegenwart von Synthesegas. Die Dialdehyde werden durch eine Umsetzung polyzyklischer aliphatischer Diene erhalten, welche mindestens zwei nicht aromatische Zyklen und zumindest zwei nicht benachbarte Doppelbindungen aufweisen. Diese Edukte können beispielsweise ein Molekulargewicht von größer oder gleich 50 g/mol und kleiner oder gleich 450 g/mol aufweisen. Neben den Doppelbindungen kann das Diolefin gegebenenfalls noch weitere funktionale Gruppen aufweisen. Mögliche Vertreter der Gruppe der einsetzbaren aliphatischen polyzyklischen Diene sind Dicyclopentadien, Tricyclopentadien, Norbornadien usw.. Der Stofftransport der erwähnten polyzyklischen aliphatischen Diolefine ist in einem heterogenen Reaktionsgemisch bestehend aus Wasser und organischer Phase limitiert. Die Homogenisierung nach dem erfindungsgemäßen Verfahren erlaubt die in Zwei-Phasen-Systemen auftretende Stofftransportlimitierung zu überwinden.

Das Synthesegas umfasst Wasserstoff und Kohlenmonoxid, wobei die Zusammensetzung des Synthesegases, d. h. das Verhältnis von Kohlenmonoxid zu Wasserstoff, variiert werden kann. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet. Je mol Diolefin kann man 1 bis 12, insbesondere 1,2 bis 10, bevorzugt 1,5 bis 8 mol Kohlenmonoxid und 2 bis 24, insbesondere 2,4 bis 20 und bevorzugt 3 bis 16 mol Wasserstoff verwenden. Es hat sich bewährt, mit einem Überschuss von 10 bis 200, insbesondere 25 bis 120 mol Kohlenmonoxid und 20 bis 400, insbesondere 50 bis 200 mol Wasserstoff je mol Diolefin zu arbeiten.

Die Umsetzung der Edukte zu den Aldehyden erfolgt an einem mit Organophosphorliganden modifizierten Metallkatalysatorsystem mit einem Übergangsmetall der 8. - 10 Nebengruppe. Der eingesetzte Katalysator oder das sich in der Reaktionslösung bildende Katalysatorsystem umfasst mindestens ein Übergangsmetall der 8. - 10. Nebengruppe des Periodensystems der Elemente wie beispielweise Cobalt, Rhodium, Iridium. Neben dem Metall weist der Katalysator zumindest einen oder mehrere organische Liganden mit einem Phosphoratom in seiner Koordinationssphäre auf oder koordiniert diese in der Reaktionslösung unter den Reaktionsbedingungen. Phosphororganische Liganden weisen dabei ein Kohlenstoffgrundgerüst mit oder ohne weitere funktionellen Gruppen und zumindest ein oder zwei kovalent gebundene Phosphoratome auf. Das katalytisch aktive Katalysatorsystem wird unter den Reaktionsbedingungen zudem durch weiteren Zutritt von Wasserstoff und Kohlenmonoxid in der Reaktionslösung gebildet, wobei die Komponenten des Synthesegases mit dem Metall einen koordinativen Komplex ausbilden. Es ist aber auch möglich, den Katalysator zunächst zu präformieren und ihn anschließend der eigentlichen Hydroformylierungsstufe zuzuführen. Die Bedingungen der Präformierung entsprechen dabei im Allgemeinen den Hydroformylierungsbedingungen.

Die Hydroformylierung wird mittels eines wasserlöslichen Triarylphosphin- oder eines wasserlöslichen Diphosphin-Komplexkatalysators durchgeführt. Der Komplexkatalysator umfasst in der Reaktionslösung in seiner Koordinationssphäre wasserlösliche organische Phosphor-Verbindungen als Liganden. Als wasserlösliche organische Phosphor-Verbindungen können beispielsweise Phosphor(III)-Verbindungen nach der allgemeinen Formel: 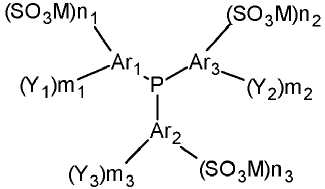 verwendet werden. In dieser Formel stehen Ar₁, Ar₂ und Ar₃ für gleiche oder verschiedene Arylgruppen mit 6 bis 14 Kohlenstoffatomen. Die Substituenten Y₁, Y₂ und Y₃ stehen für gleiche oder verschiedene geradkettige oder verzweigte Alkyl- oder Alkoxyreste mit 1 bis 4 Kohlenstoffatomen, Chlor, Brom, Hydroxyl-, Cyanid- oder Nitrogruppen, sowie ferner für Aminogruppen der Formel NR¹R², wobei die Substituenten R¹ und R² gleich oder verschieden sein können und für Wasserstoff, geradkettige oder verzweigte Alkylgruppen mit 1 bis 4 Kohlenstoffatomen stehen. Die Gegenkationen M können für Lithium, Natrium, Kalium, Magnesium, Calcium oder Barium stehen, wobei m1, m2 und m3 gleich oder verschieden sein können und ganze Zahlen von 0 bis 5 bedeuten, in der n1, n2 und n3 gleich oder verschieden sind und für ganze Zahlen von 0 bis 3 stehen, wobei mindestens eine der Zahlen n1, n2 und n3 gleich oder größer 1 ist. Ein Triarylphosphin-Komplexkatalysator ist dann ein wasserlöslicher Triarylphosphin-Komplexkatalysator, wenn die Löslichkeit des Komplexes in Wasser bei 20 °C größer oder gleich 100 g/L beträgt. Die Wasserlöslichkeit kann beispielsweise anhand der OECD-Richtlinien zur Bestimmung der Wasserlöslichkeit ("Wasserlöslichkeit" vom 27.05.1995) bestimmt werden.

Zu den wasserlöslichen Triarylphosphinen der oben angegebenen Formel zählen bevorzugt solche Triarylphosphine, bei denen die Gruppen Ar₁, Ar₂, Ar₃ Phenylgruppen sind; Y₁, Y₂ und Y₃ für eine Methyl-, eine Ethylgruppe, für eine Methoxy-, Ethoxygruppe und/oder für ein Chloratom stehen. Die kationischen Reste M der anorganischen Kationen können bevorzugt Natrium, Kalium, Calcium und Barium sein. Insbesondere können solche wasserlöslichen Triarylphosphine geeignet sein, bei denen Ar₁, Ar₂, Ar₃ jeweils für eine Phenylgruppe stehen; m1, m2, m3 gleich 0 sind, n1, n2 und n3 gleich 0 oder 1 sind und n1+n2+n3 zusammen 1 bis 3 ausmachen, wobei die Sulfonat-Gruppen bevorzugt in meta-Stellung stehen können. Die Liganden können als solche oder als Mischungen eingesetzt werden. Geeignete Beispiele für wasserlösliche Triarylphosphin-Liganden sind (Sulfophenyl)-diphenylphosphin, Di-(sulfophenyl)phenylphosphin und Tri(sulfophenylphosphin). Im Stand der Technik wird (Sulfophenyl)diphenylphosphin als TPPMS, Di-(sulfophenyl)phenylphosphin als TPPDS und Tri(sulfophenyl)phosphin als TPPTS abgekürzt.

Als wasserlösliche Diphosphine eignen sich ebenfalls sulfonierte Diphosphine der allgemeinen Formeln (III) und (IV)

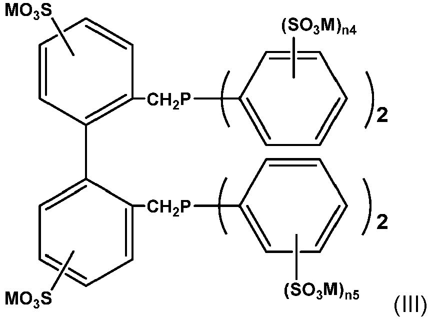

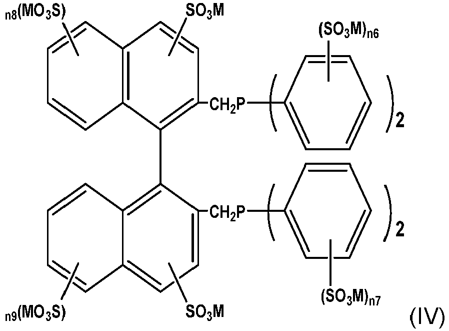

In (III) steht ein jedes n₄ und n₅ unabhängig voneinander für 0 oder 1, wobei die Verbindung der Formel (III) bis sechs -SO₃M-Gruppen enthalten kann.

In (IV) steht ein jedes n₆, n₇, n₈ und n₉ unabhängig voneinander für 0 oder 1, wobei die Verbindung der Formel (IV) vier bis acht -SO₃M-Gruppen enthält.

In den Formeln (III) und (IV) steht M für Ammonium, ein einwertiges Metall oder das Äquivalent eines mehrwertigen Metalls, insbesondere für Natrium, Kalium, Calcium oder Barium.

Die Katalysatormetall-Konzentration kann 20 bis 1000 Gew.-ppm, vorzugsweise 50 bis 800 Gew.-ppm und insbesondere 100 bis 600 Gew.-ppm bezogen auf die Gesamtmenge an wässriger Katalysatorlösung betragen. Obgleich es möglich ist, als Katalysator die stöchiometrisch zusammengesetzte Metall-Phosphorligand-Komplexverbindung einzusetzen, arbeitet man üblicherweise in Gegenwart überschüssiger Phosphor-Liganden, d.h. Ligand, der mit dem eingesetzten Metall keine komplexe Bindung eingegangen ist. Je mol Metall kann man bevorzugt 10 bis 300 mol Phosphor in Form der wasserlöslichen organischen Phosphorliganden einsetzen. Besonders bewährt haben sich molare Verhältnisse von Metall zu organischen Phosphorligand im Bereich von 1:50 bis 1:150. Der Metall-Phosphor-Komplexkatalysator muss nicht einheitlich zusammengesetzt sein, sondern kann z.B. aus einem Gemisch von Übergangsmetall-Komplexverbindungen bestehen, die sich durch die Art oder stöchiometrische Zusammensetzung der Phosphor-Liganden unterscheiden. Ebenso kann der in der wässrigen Katalysatorlösung enthaltene freie Phosphor-Ligand aus einem Gemisch unterschiedlicher wasserlöslicher organischer Phosphorverbindungen zusammengesetzt sein.

Die Hydroformylierung wird bei einem Druck von größer oder gleich 0,5 MPa und kleiner oder gleich 10 MPa und einer Temperatur von größer oder gleich 70 °C und kleiner oder gleich 150 °C durchgeführt. Die erfindungsgemäße Umsetzung erfolgt innerhalb kurzer Prozesszeiten unter für Hydroformylierungsreaktionen eher niedrigen Drücken. Dies kann helfen, die nötigen Investitionen in die Anlagentechnik zu reduzieren. Der gewählte Gesamtdruck kann sich bevorzugt über einen Bereich von 0,5 bis 10 MPa, weiterhin vorzugsweise 1 bis 8 MPa und insbesondere 1,5 bis 6 MPa erstrecken. Bevorzugt können Temperaturbereiche von 100 bis 150°C und insbesondere von 110 bis 140°C eingehalten werden.

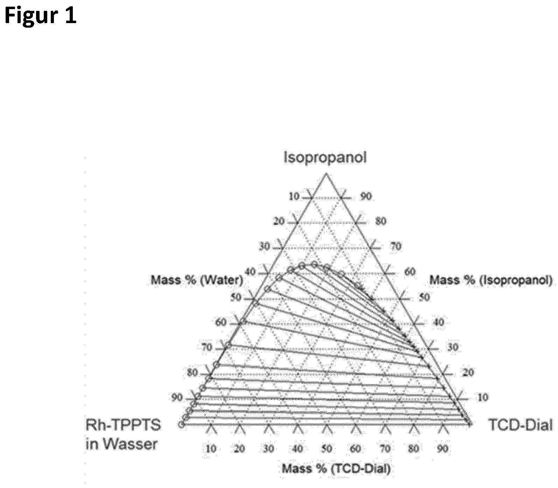

Die Reaktion wird in einer homogenen flüssigen Reaktionsphase durchgeführt, wobei die homogene flüssige Phase mindestens ein nicht-wässriges Lösungsmittel, Diolefine und/oder deren Mono- und/oder Dialdehyde als Reaktionsprodukte und eine wässrige Katalysatorlösung umfasst. Mit der gewählten Zusammensetzung aus Lösemittel, wässriger Katalysatorlösung, dem polyzyklischen Diolefin sowie den im Verlauf der Reaktion gebildeten Zwischen- sowie Endprodukten aus diesem, bildet sich am Reaktionsort innerhalb des angegebenen Druck- und Temperaturbereiches über den gesamten Reaktionsablauf eine homogene flüssige Phase aus. In dieser homogenen flüssigen Phase erfolgt der Zutritt des Synthesegases und mit diesem am Katalysator die weitere Umsetzung der ungesättigten Bindungen zu den entsprechenden Aldehyden. Dies bedeutet, dass am Reaktionsort insbesondere nicht eine zweiphasige, getrennte wässrige und organische Fraktion vorliegt, wobei die beiden Phasen eine gemeinsame Phasengrenze ausbilden. Das Vorliegen einer gemeinsamen homogenen Phase kann thermodynamisch aus den Mengenverhältnissen berechnet werden, ein Beispielergebnis der Berechnungen ist in der Figur 1 angegeben, und kann bei der Durchführung der Umsetzung visuell nachvollzogen werden. Neben den genannten Bestandteilen kann die homogene Phase auch noch überschüssige Liganden und/oder höhermolekulare Nebenprodukte aufweisen.

Die Massenverhältnisse der Edukte, des Lösungsmittels und der wässrigen Katalysatorlösung werden in der Lösung so gesteuert, dass unter den Reaktionsbedingungen eine einphasige Lösung vorliegt. Durch die Festlegung der Massenverhältnisse zwischen Edukt, sowie der wässrigen Katalysatorlösung und des Lösemittels, können unter den gewählten Temperatur- und Druckbedingungen einphasige Lösungen am Reaktionsort bereitgestellt werden, in welchen die erfindungsgemäßen Umsetzungen mit dem Synthesegas besonders vorteilhaft verlaufen. Das Steuern der Einphasigkeit bedeutet dabei, dass entweder zu Beginn der Reaktion die Massenverhältnisse der Komponenten so vorgegeben werden, dass unter den Reaktionsbedingungen eine Einphasigkeit der Lösung erreicht wird. Da zum Reaktionsstart naturgemäß noch keine Zwischen- oder Endprodukte vorliegen, müssen natürlich die Änderungen der Zusammensetzung als Funktion des Reaktionsfortschrittes berücksichtigt werden. Dazu kann beispielweise ein Punkt im Phasendiagramm, wie z.B. in der Figur 1 dargestellt, ausgesucht werden, welcher sowohl zu Anfang als auch nach vollständiger Umsetzung der Edukte im einphasigen Gebiet liegt. Es ist aber auch möglich, die Zusammensetzung während des Reaktionsverlaufes aktiv so zu steuern, dass man im einphasigen Gebiet arbeitet. Dazu kann beispielsweise Lösemittel aktiv zur Lösung gegeben oder entfernt werden. Gleiches kann auch über die wässrige Komponente erfolgen, wobei man entweder neue Katalysatorlösung oder nur Wasser zum Reaktionsort zudosiert. Weniger bevorzugt ist die prinzipiell mögliche Steuerung der Ein- oder Zweiphasigkeit über die Temperatur und den Anlagendruck.

Als Lösemittel können sich bevorzugt Flüssigkeiten anbieten, welche mit Wasser zumindest eine gewisse Löslichkeit aufweisen. Diese Löslichkeit des Lösemittels in Wasser kann bei 20°C beispielweise größer oder gleich 20 g/L betragen. Der Einsatz von Lösemitteln in diesem Löslichkeitsverhältnis zu Wasser kann dazu beitragen, dass besonders robuste einphasige Gebiete erhalten werden, welche auch möglicherweise auftretende Druck und/oder Temperaturschwankungen sicher ausgleichen können. Zudem kann der Einsatz einer solchen Gruppe an Lösemittel dazu beitragen, dass große Änderungen in der Zusammensetzung durch die Bildung von Produkten kompensiert werden können. Des Weiteren kann der Anteil der Lösemittel hinreichend klein gehalten werden, welches die Auftrennung des entstehenden Gemisches erleichtert und Energiekosten zur Abtrennung geringhält.

In einer bevorzugten Ausführungsform des Verfahrens kann das polyzyklische aliphatische Diolefin aus der Gruppe bestehend aus bi-zyklischen oder tri-zyklischen Dienen oder Mischungen daraus ausgesucht sein. Es hat sich als besonders vorteilhaft herausgestellt, dass innerhalb der erfindungsgemäßen Verfahrensführung sterisch schwierig umzusetzende zyklische Dien-Edukte mit innerständigen Doppelbindungen vollständig zu Dialdehyden umgesetzt werden, welche durch ihr rigides Ringgerüst in Lösung mit den Katalysatorkomplexen deutlich schlechter reagieren als beispielsweise kurze aliphatische Ketten. Die polyzyklischen, olefinischen Aliphaten lassen sich durch übliche Verfahrensführungen in zweiphasigen Gebieten auf Grund ihrer geringen Löslichkeit in der wässrigen Katalysatorphase nur sehr unvollständig umsetzen. Einphasige Umsetzungen in rein organischen Lösemitteln sind zwar möglich, führen aber zu erhöhten Problemen in der Aufreinigung der erhaltenen Produkte. Des Weiteren zeigen letztere Umsetzungen Probleme in der Katalysatorrückgewinnung sowie dessen Standzeiten. So z.B. werden bei Umsetzungen in rein organischen Lösungsmitteln auch unmodifizierte (ohne Zugabe von Liganden) Metall-Katalysatoren für die Umsetzung der hier genannten Diolefine eingesetzt, für welche ein hoher Katalysatoreinsatz benötigt und der Katalysator nicht rezykliert wird. Unter Einsatz von Liganden-Metall-Komplexen als Katalysatoren werden für die Hydroformylierung der polyzyklischen Diolefine oft nicht die gewünschten Aktivitäten erreicht. Die erfindungsgemäß umsetzbaren bi- oder tri-zyklischen Diene umfassen zwei oder drei geschlossene, nicht aromatische Ringe und können des Weiteren bevorzugt ein Molekulargewicht von größer oder gleich 60 g/mol und kleiner oder gleich 450 g/mol aufweisen.

In einer weiter bevorzugten Ausführungsform des Verfahrens kann das polyzyklische aliphatische Diolefin aus der Gruppe bestehend aus Dicyclopentadien oder Norbornadien ausgesucht sein. Über die erfindungsgemäße Umsetzung lassen sich insbesondere sterisch schwierig umzusetzende sowie schlecht wasserlösliche polyzyklische aliphatische Olefine wie das Tricyclo[5.2.1.02,6]deca-3,8-dien und das Bicyclo[2.2.1]hepta-2,5-dien besonders effizient umsetzen. Es werden hohe Umsätze bei hohen Selektivitäten erzielt, wobei das Katalysatorsystem auch eine besonders lange Standzeit aufweisen kann.

In einer bevorzugten Ausgestaltung des Verfahrens kann die Temperatur und der Druck während der Umsetzung konstant gehalten und die Einphasigkeit der Reaktionslösung über die Massenverhältnisse von Lösungsmittel zur wässrigen Katalysatorlösung eingestellt werden. Es hat sich zudem als vorteilhaft herausgestellt, dass der gewünschte einphasige Bereich im Wesentlichen über die Massenverhältnisse zwischen wässriger Katalysatorlösung, dem Edukt und dem Lösemittel eingestellt wird. Es können hier "sichere" Bereiche erhalten werden, so dass die Veränderung der Zusammensetzung im Laufe der Reaktion durch die Bildung der Zwischen- und/oder Endprodukte, die Bildung höhermolekularer Nebenprodukte und/oder eine Veränderung der organischen Phosphorliganden sicher kompensiert werden können. Zudem kann über die Wahl der geeigneten Lösemittel und dessen Menge im Verhältnis zur wässrigen Katalysatorlösung auch eventuell auftretende Temperatur- und/oder Druckschwankungen kompensiert werden. Über diese Festlegung lässt sich die Reaktion in ihrer Gesamtheit sicher im einphasigen Bereich halten. Weiter bevorzugt kann diese Steuerung über die Wahl der Mengen an Lösemittel und wässriger Katalysatorlösung zu Beginn der Reaktion festgelegt werden.

In einer weiter bevorzugten Charakteristik des Verfahrens kann das Lösungsmittel aus der Gruppe bestehend aus geradkettigen oder verzweigten C2-C5 Alkoholen oder Mischungen mindestens zweier Alkohole aus dieser Gruppe ausgesucht sein. Insbesondere die kurzkettigen Alkohole haben sich zum Erhalt besonders effizienter Umsetzungen in einem einphasigen Gebiet als besonders geeignet herausgestellt. Mittels dieser Lösemittel lassen sich auch schwierig zu hydroformylierende Edukte innerhalb sehr kurzer Prozesszeiten an wasserlöslichen Katalysatoren hoch selektiv umsetzten. Auch können die Standzeiten der Katalysatoren durch diese Lösemittelwahl deutlich verlängert werden. Diese Lösemittel zeigen im Vergleich zum Liganden eine geringe Bindungsaffinität zum Metall. Zugleich können sie sich aber stabilisierend auf den Liganden und damit vor Abbau schützend auswirken. Ein weiterer Vorteil ergibt sich dadurch, dass mit nur einem geringen Anteil an Lösemitteln sehr robuste einphasige Gebiete erreicht werden können, welches die Kosten der Aufarbeitung und Abtrennung der gewünschten Produkte reduziert. Vorteilhaft sind auch die niedrigen Siedepunkte der ausgewählten Lösemittel, wodurch sie leicht vom Reaktionssystem abgetrennt werden können.

Innerhalb eines bevorzugten Aspektes des Verfahrens kann das Lösungsmittel Isopropanol sein. Die Verwendung von Isopropanol zur einphasigen Umsetzung polyzyklischer Diene im Rahmen einer Hydroformylierung hat sich als besonders günstig herausgestellt. Es lassen sich durch Isopropanolzusatz auch schwierig zu hydroformylierende Edukte innerhalb sehr kurzer Prozesszeiten an wasserlöslichen Katalysatoren hoch selektiv umsetzen. Auch können die Standzeiten der Katalysatoren durch dieses Lösemittel deutlich verlängert werden. Ein weiterer Vorteil ergibt sich dadurch, dass mit nur einem geringen Isopropanolanteil sehr robuste einphasige Gebiete ausgebildet werden, welches die Kosten der Aufarbeitung und Abtrennung der gewünschten Produkte reduziert. Zudem kann durch die physikalischen Unterschiede des Isopropanols zu den Aldehyd-Produkten eine besonders einfache und vollständige Trennung nach Reaktionsende erreicht werden.

Im Rahmen einer bevorzugten Ausgestaltung des Verfahrens kann der Metallkatalysator in einer Konzentration von größer oder gleich 0,05 mol und kleiner oder gleich 0,75 mol in der Reaktionslösung vorliegen. Über die einphasige Umsetzung können auch effiziente Verfahrensführungen erreicht werden, welche mit deutlich geringeren Katalysatormengen auskommen. Mit diesen geringen Gehalten lassen sich innerhalb kurzer Reaktionszeiten hoch selektive Umsetzungen erreichen. Ohne durch die Theorie gebunden zu sein, wird dies hochwahrscheinlich auch dadurch erreicht, dass über die einphasige Reaktionsführung ein stärkerer Abbau der Katalysatorliganden und damit auch des Katalysatorsystems selbst, verhindert oder doch zumindest gemindert wird. Des Weiteren erreicht man über die Modifizierung der Polarität der Lösung einen besonders effizienten Zutritt des Synthesegases, welches zu besonders schnellen Reaktionen beitragen kann. Ohne durch die Theorie gebunden zu sein, kann eine Mischung aus Wasser und Isopropanol auch im besonderen Maße geeignet sein, um einen vorzeitigen Abbau der wasserlöslichen, organischen Liganden bei sehr gering eingesetzten Katalysatorkonzentrationen zu verhindern. Bevorzugt kann der Katalysator in der Reaktionslösung in einer Konzentration von größer oder gleich 0,1 mol/L und kleiner oder gleich 0,65 mol/L, des Weiteren bevorzugt von größer oder gleich 0,2 mol/L und kleiner oder gleich 0,55 mol/L vorliegen.

Innerhalb eines weiter bevorzugten Aspektes des Verfahrens kann die Reaktion bei einem Druck von größer oder gleich 2,5 MPa und kleiner oder gleich 7,5 MPa, bevorzugt von größer oder gleich 3 MPa bis kleiner oder gleich 7 MPa und insbesondere von größer oder gleich 3,5 MPa bis kleiner oder gleich 6,5 MPa durchgeführt werden. Überraschend hat sich zur Umsetzung schwieriger Edukte zudem eine Reaktionsführung in einem besonders niedrigen Druckbereich als besonders günstig herausgestellt. Trotz der sterisch herausfordernden Edukte, deren Schwerlöslichkeit und der gewünschten vollständigen Umsetzung beider Doppelbindungen, scheint die wässrige Komponente im einphasigen Gebiet nicht den Zutritt des Synthesegases zu hemmen, sodass insgesamt schon geringere Drücke für einen ausreichenden Gaseintrag in die Reaktionslösung sorgen können.

Innerhalb einer bevorzugten Ausführungsform des Verfahrens kann das Massenverhältnis von wässriger Katalysatorlösung zu Lösungsmittel, ausgedrückt als Masse Katalysatorlösung dividiert durch Masse Lösungsmittel, größer oder gleich 0,25 und kleiner oder gleich 4 betragen. In der gesamten einphasigen Reaktionslösung hat sich oben angegebenes Massenverhältnis als besonders sicher und vorteilhaft herausgestellt. Über dieses Massenverhältnis lassen sich auch verfahrensbedingte, unvermeidliche Schwankungen des Drucks und der Temperatur, sowie die Änderung der Zusammensetzung durch die Bildung der Produkte sicher kompensieren. Weiterhin vorteilhaft kann sich eine, bezogen auf die Masse der Katalysatorlösung, relativ geringe Menge an Lösemittelzusatz von größer oder gleich 0,5 und kleiner oder gleich 2, insbesondere aber größer oder gleich 0,75 und kleiner oder gleich 1,5 ergeben. Dieser geringe Lösemittelzusatz kann zu einer effizienteren Aufarbeitung der Reaktionslösung nach Reaktionsende beitragen.

Im Rahmen eines bevorzugten Aspektes des Verfahrens kann die Reaktion bei einer Temperatur von größer oder gleich 120 °C und kleiner oder gleich 150 °C durchgeführt werden. Trotz der gesteigerten Reaktivität durch das Fahren im einphasigen Bereich, hat sich oben angegebenes Temperaturintervall als vorteilhaft herausgestellt, insbesondere bei einer Temperatur von größer oder gleich 125 °C und kleiner oder gleich 140 °C. Es werden in diesem Bereich besonders schnelle und selektive Umsetzungen erhalten, wobei insbesondere auch der Anteil an gebildeten Hochsiedern sehr niedrig bleibt. Zudem ergeben sich deutlich verlängerte Katalysatorstandzeiten, welches wahrscheinlich auf einem verringerten Abbau der organischen Liganden zurückgeführt werden kann.

In einer weiter bevorzugten Ausführungsform des Verfahrens kann der Komplexkatalysator Triarylphosphin-Liganden und ein Katalysatormetall umfassen, wobei das molare Einsatzverhältnis von Triarylphosphin-Liganden zu Katalysatormetall, ausgedrückt als mol Triarylphosphin-Liganden dividiert durch mol Katalysatormetall, größer oder gleich 3 und kleiner oder gleich 15 beträgt. Für die Umsetzungen im einphasigen Bereich hat es sich als vorteilhaft herausgestellt, dass das Verhältnis von organischen Liganden zu Katalysatormetall in einem engen Bereich, bevorzugt größer oder gleich 5 und kleiner oder gleich 12 und insbesondere größer oder gleich 7 und kleiner oder gleich 10 gehalten wird. Innerhalb dieser Verhältnisse ergeben sich sehr reproduzierbare Umsetzungen mit hohen Selektivitäten. Dies ist wahrscheinlich deshalb möglich, da eine geringere Liganden-Konzentration höhere Aktivitäten des Katalysators erlaubt. Zugleich wird der Abbau des organischen Liganden in der einphasigen Lösung verzögert oder sogar ganz verhindert. Somit kann diese Verfahrensführung dazu beitragen, dass der Katalysator häufiger und länger verwendet werden kann.

In einer weiterhin bevorzugten Ausgestaltung des Verfahrens kann das Synthesegas ein molares CO zu H₂ Verhältnis, ausgedrückt als mol CO dividiert durch mol H₂, von größer oder gleich 0,5 und kleiner oder gleich 2 aufweisen. Durch den verbesserten Zutritt des Synthesegases in die homogene einphasige Reaktionslösung kann man im Rahmen dieser Umsetzung besonders vorteilhaft mit oben genannter Synthesegaszusammensetzung fahren. Üblicherweise werden andere Verhältnisse eingesetzt, welches der Tatsache Rechnung trägt, dass CO innerhalb einer zweiphasigen Verfahrensführung schlechter zwischen den unterschiedlichen Phasen diffundieren kann. Somit kann neben der Erhöhung der Reaktionsgeschwindigkeit auch sehr ressourceneffizient gearbeitet werden.

Innerhalb einer weiteren Ausführungsform des Verfahrens kann der pH-Wert der wässrigen Katalysatorlösung größer oder gleich pH 4 und kleiner oder gleich pH 10 betragen. Es hat sich herausgestellt, dass durch Einstellen des pH-Wertes in dem bevorzugten Bereich der Katalysator aus dem Übergangsmetall und dem wasserlöslichen Organophosphorliganden sehr hohe Aktivität und hohe Selektivität bezüglich der Produktbildung aufweist. Die Einstellung kann durch bekannte Stellmittel wie anorganische Säuren oder Basen an der eingesetzten Katalysatorlösung erfolgen. Es ist aber auch möglich und vorteilhaft, dass die mittels der eingesetzten wässrigen Katalysatorlösung sich ausbildende homogene Phase auf den beschriebenen pH-Wertebereich gehalten wird. Des Weiteren wurde bei der bevorzugten pH-Wert-Einstellung niedrige Zersetzung des Katalysators beobachtet. In einer weiter bevorzugten Ausführungsform kann der pH-Wert zwischen größer oder gleich pH 5 und kleiner oder gleich pH 8, des Weiteren bevorzugt zwischen größer oder gleich pH 5,5 und kleiner oder gleich pH 7 eingestellt werden.

In einer bevorzugten Ausführungsform des Verfahrens kann das molare Verhältnis von Wasser in der eingesetzten wässrigen Katalysatorlösung zu Katalysatormetall, ausgedrückt als mol Wasser dividiert durch mol Katalysatormetall, größer oder gleich 5000 und kleiner oder gleich 60000 betragen. Trotz der Tatsache, dass der Zutritt der organischen Edukte an den Katalysator durch ein eher organisches Umfeld verbessert werden sollte, hat sich oben angegebenes Verhältnis von Wasser und Katalysator als besonders günstig herausgestellt. Mit diesen Wasseranteilen werden für Dien-Edukte vollständige Umsetzungen zu den Dialdehyden erreicht und die Verfahrensführung kann auch sicher einphasig ausgestaltet werden. Zusätzlich können Schwankungen der Reaktionsbedingungen sicher ausgeglichen werden, ohne dass das einphasige Phasengebiet verlassen wird.

In einer weiter bevorzugten Charakteristik des Verfahrens kann das molare Verhältnis von Katalysatormetall zu polyzyklischem aliphatischen Diolefin, ausgedrückt als mol Katalysatormetall zu mol Diolefin, größer oder gleich 0,05 % und kleiner oder gleich 0,75 % , bevorzugt größer oder gleich 0,15 % und kleiner oder gleich 0,65 %, insbesondere größer oder gleich 0,3 % und kleiner oder gleich 0,5% betragen. Durch die einphasige Reaktionsführung kann auch sehr effizient mit einem besonders geringen Verhältnis von Katalysator zu Olefin-Edukt gefahren werden. Es werden innerhalb kurzer Reaktionszeiten vollständige Umsetzungen zu den Dialdehyden erreicht, wobei auch die KatalysatorStandzeiten im Vergleich zu den Stand-der-Technik-Lösungen länger sein können.

Innerhalb einer bevorzugten Ausgestaltung des Verfahrens kann mindestens ein Ligand des wasserlöslichen Triarylphosphin-Komplexkatalysators ein Triphenylphosphin-3,3',3"-trisulfonsäure Natriumsalz umfassen. Der Einsatz dieser Triphenylphosphin-Liganden hat sich für das Arbeiten im einphasigen Bereich als besonders vorteilhaft herausgestellt. Neben einer hochselektiven Umsetzung zu den Dialdehyden werden, auch bei langen Reaktionszeiträumen, besonders geringe Mengen an Hochsiedern gebildet. Dies ergibt sich insbesondere bei dem Einsatz von Isopropanol als Lösungsmittel, wobei in diesen Fällen sich ein besonders geringer Abbau des organischen Liganden einstellt.

### Beispiele

In einer erfindungsgemäßen Hydroformylierung wird Dicyclopentadien DCDP mittels eines organisch modifizierten Rhodium-Komplexkatalysators in einer Reaktionslösung zu den entsprechenden Dialaldehyd nach folgender Reaktionsgleichung: 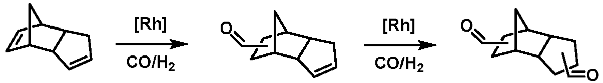 umgesetzt. Als Katalysator wird ein wasserlöslicher Komplexkatalysator eingesetzt, welcher phosphororganischen Liganden nach folgender Formel umfasst:

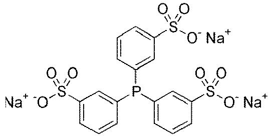

Als Lösungsmittel zur Erreichung der Einphasigkeit der Reaktionssystems wird Isopropanol verwendet. Die Umsetzung erfolgt bei 130 °C und einem Druck von 5 MPa in einem gerührten Reaktorkessel (800 U/min) innerhalb einer Reaktionszeit von 3 h.

Die Einsatzmengen der Edukte ergeben sich wie folgt:

| **Bestandteil** | **Einsatzmenge** |
|---|---|
| Gesamt-Rhodium Konzentration in wässriger Katalysatorlösung | 46380 mg/l |
| Menge wässrige Katalysatorlösung | 2,32 ml |
| Rhodium in mol | 1,04387 mmol |
| Ligand in g | 18,38 g |
| Ligand in mol | 568 mmol |
| P/Rh-Verhältnis | 10:1 |
| Olefin in % bezogen auf gesamten Einsatz | 10% |
| Olefin in g | 50,0 g |
| Olefin in mol | 0,378 mol |
| Rhodiummenge bezogen auf Olefin | 0,46% |
| Einsatzmenge Isopropanol | 135 g |
| Einsatzmenge Wasser | 135 g |
| Synthesegaszusammensetzung H₂:CO | 1:1 |

Es wurde eine wässrige Katalysatorlösung aus Rhodium und dem Liganden hergestellt. Als Rhodiumquelle wurde Rh(OAc)₂ eingesetzt. Diese Lösung wurde mit der oben angegebenen Menge Isopropanol in den Reaktor gegeben. Dicyclopentadien wurde zudosiert und bei 5 MPa Synthesegas Druck und 130 °C für 3 h zur Reaktion gebracht. Nach Abkühlen und Entspannen des Autoklavs wurde das Isopropanol vom Reaktionsgemisch bei 100 mbar und 40 °C entfernt. Nachfolgend wurde der Rückstand in einen Phasentrenner gegeben, wodurch die Separation der Katalysator- und Produktphase ermöglicht wurde. Die Produktphase wurde mittels GC untersucht. Es ergab sich folgende Zusammensetzung:

| **Bestandteil (ohne Lösemittel)** | **Flächen-%** |
|---|---|
| Vorlauf | 0,48 |
| DCP | 1,50 |
| TCD-Monoenal-Isomere | 3,38 |
| TCD-Dial-Isomere | 89,92 |
| TCD-OH | 2,20 |
| Nachlauf | 2,52 |

Die Ergebnisse ergeben sich ohne Berücksichtigung des Lösemittelanteils. Die Ergebnisse zeigen, dass mit einer sehr geringen Katalysatorkonzentration von 0,45 mol-% bezogen auf das Diolefin ein fast 100%iger Umsatz erreicht wurde. Zudem ergab sich eine TCD Dial-Selektivität von ca. 90%. Die eingesetzte wässrige Katalysatorlösung konnte in mehreren Versuchen (n>5) ohne Verlust der Reaktivität eingesetzt werden. Dies ist ein Indiz dafür, dass das erfindungsgemäße Verfahren den Katalysator anscheinend auch vor der Zersetzung schützen kann.

Die Figur 1 zeigt das Ergebnis des berechneten Phasenverhaltens eines drei Komponenten-Gemisches als Funktion der Zusammensetzung bei einer Temperatur von 120°C. Als Komponenten liegen die wässrige Lösung eines Rh-TPPTS-Komplexkatalysators (unten links), Isopropanol als Lösungsmittel (oben) und das Endprodukt TCD-Dial (unten rechts) vor. Der gestrichelte Bereich umfasst die Massenanteile der ternären Zusammensetzungen, welche bei dieser Temperatur zwei-phasig vorliegen. Im nicht-gestrichelten, oberen Bereich des Dreiecks liegen Zusammensetzungen vor, welche unter den Druck- und Temperaturbedingungen einphasig sind. Da sich die Löslichkeiten der Olefin-Edukte nicht signifikant von den Löslichkeiten der Zwischen- und Endprodukte unterscheiden, ist dieses Phasendiagramm für die gesamte Umsetzung mit wechselnden Edukt-/Produkt-Verhältnissen repräsentativ.

## Patentansprüche

1. Verfahren zur Herstellung polyzyklischer aliphatischer Dialdehyde durch Hydroformylierung polyzyklischer aliphatischer Diolefine in Gegenwart von Synthesegas an einem mit Organophosphorliganden modifizierten Metallkatalysatorsystem mit einem Übergangsmetall der 8. - 10. Nebengruppe, **dadurch gekennzeichnet, dass** die Hydroformylierung mittels eines wasserlöslichen Diphosphin- oder Triarylphosphin-Komplexkatalysators bei einem Druck von größer oder gleich 0,5 MPa und kleiner oder gleich 10 MPa und einer Temperatur von größer oder gleich 70 °C und kleiner oder gleich 150 °C in einer homogenen flüssigen Reaktionsphase durchgeführt wird, wobei die homogene flüssige Phase mindestens ein nicht-wässriges Lösungsmittel, Olefine und/oder deren Mono- und/oder Dialdehyde als Reaktionsprodukte und eine wässrige Katalysatorlösung umfasst, wobei die Massenverhältnisse dieser Komponenten in der Lösung so gesteuert werden, dass unter den Reaktionsbedingungen eine einphasige Lösung vorliegt.

2. Verfahren nach Anspruch 1, wobei das polyzyklische aliphatische Diolefin ausgesucht ist aus der Gruppe bestehend aus bi-zyklischen oder tri-zyklischen Dienen oder Mischungen daraus.

3. Verfahren nach einem der vorhergehenden Ansprüche, wobei das polyzyklische aliphatische Diolefin aus der Gruppe bestehend aus Dicyclopentadien oder Norbornadien ausgesucht ist.

4. Verfahren nach einem der vorhergehenden Ansprüche, wobei die Temperatur und der Druck während der Umsetzung konstant gehalten werden und die Einphasigkeit der Reaktionslösung über die Massenverhältnisse von Lösungsmittel zur wässrigen Katalysatorlösung eingestellt werden.

5. Verfahren nach einem der vorhergehenden Ansprüche, wobei das Lösungsmittel aus der Gruppe bestehend aus geradkettigen oder verzweigten C2-C5 Alkoholen oder Mischungen mindestens zweier Alkohole aus dieser Gruppe ausgesucht ist.

6. Verfahren nach einem der vorhergehenden Ansprüche, wobei das Lösungsmittel Isopropanol ist.

7. Verfahren nach einem der vorhergehenden Ansprüche, wobei der Metallkatalysator in einer Konzentration von größer oder gleich 0,05 mol und kleiner oder gleich 0,75 mol in der Reaktionslösung vorliegt.

8. Verfahren nach einem der vorhergehenden Ansprüche, wobei die Reaktion bei einem Druck von größer oder gleich 2,5 MPa und kleiner oder gleich 7,5 MPa durchgeführt wird.

9. Verfahren nach einem der vorhergehenden Ansprüche, wobei das Massenverhältnis von wässriger Katalysatorlösung zu Lösungsmittel, ausgedrückt als Masse Katalysatorlösung dividiert durch Masse Lösungsmittel, größer oder gleich 0,25 und kleiner oder gleich 4 beträgt.

10. Verfahren nach einem der vorhergehenden Ansprüche, wobei die Reaktion bei einer Temperatur von größer oder gleich 120 °C und kleiner oder gleich 150 °C durchgeführt wird.

11. Verfahren nach einem der vorhergehenden Ansprüche, wobei der Komplexkatalysator Triarylphosphin-Liganden und ein Katalysatormetall umfasst, wobei das molare Einsatzverhältnis von Triarylphosphin-Liganden zu Katalysatormetall, ausgedrückt als mol Triarylphosphin-Liganden dividiert durch mol Katalysatormetall, größer oder gleich 8 und kleiner oder gleich 15 beträgt.

12. Verfahren nach einem der vorhergehenden Ansprüche, wobei der pH-Wert der wässrigen Katalysatorlösung größer oder gleich pH 4 und kleiner oder gleich pH 10 beträgt.

13. Verfahren nach einem der vorhergehenden Ansprüche, wobei das molare Verhältnis von Wasser in der eingesetzten wässrigen Katalysatorlösung zu Katalysatormetall, ausgedrückt als mol Wasser dividiert durch mol Katalysatormetall, größer oder gleich 5000 und kleiner oder gleich 60000 beträgt.

14. Verfahren nach einem der vorhergehenden Ansprüche, wobei das molare Verhältnis von Katalysatormetall zu polyzyklischem aliphatischen Diolefin, ausgedrückt als mol Katalysatormetall zu mol Diolefin, größer oder gleich 0,05 % und kleiner oder gleich 0,75 % beträgt.

15. Verfahren nach einem der vorhergehenden Ansprüche, wobei mindestens ein Ligand des wasserlöslichen Triarylphosphin-Komplexkatalysators ein Triphenylphosphin-3,3',3"-trisulfonsäure Natriumsalz umfasst.

## Claims

1. Process for the preparation of polycyclic aliphatic dialdehydes by hydroformylation of polycyclic aliphatic diolefins in the presence of synthesis gas over a metal catalyst system modified with organophosphorus ligands with a transition metal of the 8^{th}-10^{th} subgroup, **characterized in that** the hydroformylation is carried out by means of a water-soluble diphosphine or triarylphosphine complex catalyst at a pressure of greater than or equal to 0.5 MPa and less than or equal to 10 MPa and at a temperature of greater than or equal to 70°C and less than or equal to 150°C in a homogeneous liquid reaction phase, the homogeneous liquid phase comprising at least one non-aqueous solvent, olefins and/or their mono- and/or dialdehydes as reaction products and an aqueous catalyst solution, the mass ratios of these components in the solution being controlled so as to obtain a single-phase solution under the reaction conditions.

2. The process according to claim 1, wherein the polycyclic aliphatic diolefin is selected from the group consisting of bi-cyclic or tri-cyclic dienes or mixtures thereof.

3. The process according to any one of the preceding claims, wherein the polycyclic aliphatic diolefin is selected from the group consisting of dicyclopentadiene or norbornadiene.

4. The process according to any one of the preceding claims, wherein the temperature and the pressure are kept constant during the reaction and the single phase of the reaction solution is adjusted via the mass ratios of solvent to aqueous catalyst solution.

5. The process according to any one of the preceding claims, wherein the solvent is selected from the group consisting of straight chain or branched C2-C5 alcohols or mixtures of at least two alcohols from this group.

6. The process according to any one of the preceding claims, wherein the solvent is isopropanol.

7. The process according to any one of the preceding claims, wherein the metal catalyst is present in a concentration of greater than or equal to 0.05 mol and less than or equal to 0.75 mol in the reaction solution.

8. The process according to any one of the preceding claims, wherein the reaction is carried out at a pressure of greater than or equal to 2.5 MPa and less than or equal to 7.5 MPa.

9. The process according to any one of the preceding claims, wherein the mass ratio of aqueous catalyst solution to solvent, expressed as mass of catalyst solution divided by mass of solvent, is greater than or equal to 0.25 and less than or equal to 4.

10. The process according to any one of the preceding claims, wherein the reaction is carried out at a temperature greater than or equal to 120 °C and less than or equal to 150 °C.

11. The process according to any one of the preceding claims, wherein the complex catalyst comprises triarylphosphine ligands and a catalyst metal, wherein the molar ratio of triarylphosphine ligands to catalyst metal, expressed as moles of triarylphosphine ligands divided by moles of catalyst metal, is greater than or equal to 8 and less than or equal to 15.

12. The process according to any one of the preceding claims, wherein the pH of the aqueous catalyst solution is greater than or equal to pH 4 and less than or equal to pH 10.

13. The process according to any one of the preceding claims, wherein the molar ratio of water in the used aqueous catalyst solution to catalyst metal, expressed as moles of water divided by moles of catalyst metal, is greater than or equal to 5000 and less than or equal to 60000.

14. The process according to any one of the preceding claims, wherein the molar ratio of catalyst metal to polycyclic aliphatic diolefin, expressed as moles of catalyst metal to moles of diolefin, is greater than or equal to 0.05% and less than or equal to 0.75%.

15. The process according to any one of the preceding claims, wherein at least one ligand of the water-soluble triarylphosphine complex catalyst comprises a triphenylphosphine-3,3',3"-trisulfonic acid sodium salt.

## Revendications

1. Procédé de fabrication de di aldéhydes aliphatiques polycycliques par hydroformylation de di oléfines aliphatiques polycycliques en présence d'un gaz de synthèse sur un système de catalyseur métallique modifié avec des ligands organophosphorés avec un métal de transition du groupe secondaire 8 à 10, **caractérisé en ce que** l'hydroformylation est effectuée au moyen d'un catalyseur complexe de di phosphine ou de tri aryl phosphine soluble dans l'eau à une pression égale ou supérieure à 0,5 MPa et inférieure ou égale à 10 MPa, et une température égale ou supérieure à 70 °C et inférieure ou égale à 150 °C dans une phase réactionnelle liquide homogène, où la phase liquide homogène comprend au moins un solvant non aqueux, des oléfines et/ou leurs mono et/ou leurs di aldéhydes en tant que produits de réaction et une solution de catalyseur aqueuse, où les rapports massiques de ces composants dans la solution sont contrôlés de telle manière qu'une solution monophasique est présente dans les conditions réactionnelles.

2. Procédé selon la revendication 1, dans lequel la di oléfine aliphatique polycyclique est choisie dans le groupe constitué de diènes bi-cycliques ou tri-cycliques ou de mélanges de ceux-ci.

3. Procédé selon l'une des revendications précédentes, dans lequel la di oléfine aliphatique polycyclique est choisie dans le groupe constitué du di cyclopentadiène ou de norbornadiène.

4. Procédé selon l'une des revendications précédentes, dans lequel la température et la pression sont maintenues constantes pendant la conversion et le caractère monophasique de la solution réactionnelle est contrôlé par le biais des rapports massiques du solvant sur la solution de catalyseur aqueuse.

5. Procédé selon l'une des revendications précédentes, dans lequel le solvant est choisi dans le groupe constitué d'alcools linéaires ou ramifiés en C2 à C5 ou de mélanges d'au moins deux alcools de ce groupe.

6. Procédé selon l'une des revendications précédentes, dans lequel le solvant est l'isopropanol.

7. Procédé selon l'une des revendications précédentes, dans lequel le catalyseur métallique est présent dans une concentration égale ou supérieure à 0,05 mole et inférieure ou égale à 0,75 mole dans la solution réactionnelle.

8. Procédé selon l'une des revendications précédentes, dans lequel la réaction est effectuée à une pression égale ou supérieure à 2,5 MPA et inférieure ou égale à 7,5 MPa.

9. Procédé selon l'une des revendications précédentes, dans lequel le rapport massique de la solution de catalyseur sur le solvant, exprimé sous la forme de la masse de solution de catalyseur divisée par la masse de solvant, est égal ou supérieur à 0,25 et inférieur ou égal à 4.

10. Procédé selon l'une des revendications précédentes, dans lequel la réaction est effectuée à une température égale ou supérieure à 120 °C et inférieure ou égale à 150 °C.

11. Procédé selon l'une des revendications précédentes, dans lequel le catalyseur complexe comprend des ligands tri arylphosphine et un métal catalyseur, dans lequel le rapport de l'engagement molaire des ligands tri arylphosphine sur le métal catalyseur, exprimé sous forme de moles de ligands de tri arylphosphine divisées par les moles de métal catalyseur, est égal ou supérieur à 8 et inférieur ou égal à 15.

12. Procédé selon l'une des revendications précédentes, dans lequel la valeur du pH de la solution de catalyseur aqueuse est égale ou supérieure à pH 4 et inférieure ou égale à pH 10.

13. Procédé selon l'une des revendications précédentes, dans lequel le rapport molaire de l'eau dans la solution de catalyseur aqueuse engagée sur le métal catalyseur, exprimé sous la forme des moles d'eau divisées par les moles de métal catalyseur, est égal ou supérieur à 5000 et inférieur ou égal à 60000.

14. Procédé selon l'une des revendications précédentes, dans lequel le rapport molaire du métal catalyseur sur les di oléfines aliphatiques polycycliques, exprimé sous la forme des moles de métal catalyseur sur les moles de di oléfines, est égal ou supérieur à 0,05 % et inférieur ou égal à 0,75 %.

15. Procédé selon l'une des revendications précédentes, dans lequel au moins un ligand du catalyseur complexe tri arylphosphine soluble dans l'eau comprend un sel de sodium d'acide tri phényl phosphine-3,3',3"-trisulfonique.
